# EUROPÄISCHE PATENTSCHRIFT

(11) **EP 1 842 510 B1**
(45) Veröffentlichungstag und Bekanntmachung des Hinweises auf die Patenterteilung: **03.09.2008**
(21) Anmeldenummer: 07004640.4
(22) Anmeldetag: 07.03.2007
(51) Int. Cl.: A61F 2/44

(54) **Zwischenwirbelimplantat**
Intervertebral implant
Implant intervertébral

(30) Priorität: 06.04.2006 DE 102006016986
(43) Veröffentlichungstag der Anmeldung: 10.10.2007
(73) Patentinhaber: AESCULAP AG, 78532 Tuttlingen (DE)
(72) Erfinder: Beger, Jens, 78532 Tuttlingen (DE); Wegmann, Jürgen, 78333 Stockach (DE)
(74) Vertreter: Hoeger, Stellrecht & Partner Patentanwälte

(56) Entgegenhaltungen:
- EP-A1- 0 610 837
- US-A- 5 314 478
- US-A1- 2004 068 320

## Beschreibung

Die Erfindung betrifft ein Zwischenwirbelimplantat mit mindestens einem eine Wirbelkörper-Anlagefläche aufweisenden Anlageelement und mit einem quellfähigen Kern, der auf der von der Wirbelkörper-Anlagefläche abgewandten Seite mit dem Anlageelement verbunden ist, wobei das Anlageelement einen Träger umfaßt, auf dessen einer Seite die Wirbelkörper-Anlagefläche angeordnet ist und der auf seiner anderen Seite Vorsprünge trägt, die von dem Material des Kerns umschlossen werden.

Für die Rekonstruktion der Bandscheibe werden Zwischenwirbelimplantate eingesetzt, die einen zwischen zwei Endplatten aus Metall oder Kunststoff angeordneten Hydrogelkern aufweisen. Dieser Hydrogelkern besteht aus einem quellfähigen Material, das unter Aufnahme von Wasser sein Volumen vergrößern kann und daher eine gewisse strukturelle Ähnlichkeit zu einer natürlichen Bandscheibe aufweist. Die dauerhafte Verbindung zwischen einem solchen Kern aus einem relativ weichen Material einerseits und den Endplatten aus einem starren Material andererseits ist schwierig zu bewerkstelligen. Es ist beispielsweise bekannt, das quellfähige Material in eine poröse Struktur der Endplatten einwandern zu lassen (US 5,314,478), dies ist jedoch nur dann möglich, wenn die Endplatten eine entsprechende poröse Struktur aufweisen. Außerdem können sich dabei Probleme dadurch ergeben, daß die poröse Struktur durch das quellfähige Material soweit ausgefüllt wird, daß das Einwachsen von Knochensubstanz in die poröse Struktur behindert wird.

Es sind auch Konstruktionen von Zwischenwirbelkörpern bekannt, bei denen weiche Kernmaterialien über Vorsprünge mit den Endplatten verbunden werden, diese Vorsprünge sind dabei als Rippen oder Leisten ausgebildet (US 5,674,294) oder als einschraubbare Teller (EP 0317972 A1). Die Verbindung erfolgt dabei nur immer in einem Teilbereich der Kontaktfläche zwischen Kernmaterial und dem Material der Endplatten oder Anlageelemente.

Es ist Aufgabe der Erfindung, ein gattungsgemäßes Zwischenwirbelimplantat so zu verbessern, daß der Kontakt zwischen einem weicheren Kernmaterial einerseits und einem daran anliegenden Anlageelement des Zwischenwirbelimplantats andererseits verbessert wird.

Diese Aufgabe wird bei einem Zwischenwirbelimplantat der eingangs beschriebenen Art erfindungsgemäß dadurch gelöst, daß die Vorsprünge längliche faden- oder stabförmige Verankerungsabschnitte aufweisen, die im Abstand vom Träger verlaufen und allseits frei zugänglich sind. Das weiche Material umgibt diese faden- oder stabförmigen Abschnitte aufgrund ihrer allseits freien Zugänglichkeit allseitig, die Verankerungsabschnitte sind also vollständig in das weichere Material eingebettet und bilden so ein Zwischenglied zwischen dem Träger und dem Kernmaterial, das diese Materialien dauerhaft und sehr fest miteinander verbindet.

Die Verankerungsabschnitte verlaufen vorzugsweise im wesentlichen parallel zur Wirbelkörper-Anlagefläche und im Abstand von der der Wirbelkörper-Anlagefläche abgewandten Seite des Trägers, so daß zwischen den Verankerungsabschnitten und dem Träger eine geschlossene Schicht des weicheren Kernmaterials angeordnet sein kann.

Besonders vorteilhaft ist es, wenn im wesentlichen die gesamte Fläche des Trägers Verankerungsabschnitte aufweist. Es kann dann eine Verbindung zwischen dem Träger einerseits und dem Kernmaterial andererseits über die gesamte Kontaktfläche der beiden Materialien hergestellt werden, die Verankerung wird also nicht nur auf bestimmte Bereiche der Kontaktfläche beschränkt.

Mehrere Verankerungsabschnitte können parallel zueinander verlaufen.

Bei einer besonders bevorzugten Ausführungsform ist vorgesehen, daß sich verschiedene Verankerungsabschnitte überkreuzen.

Insbesondere können sich überkreuzende Verankerungsabschnitte eine netzförmige Struktur ausbilden, also eine Struktur nach Art eines Gewebes oder Gewirkes.

Besonders vorteilhaft ist es, wenn der Träger rahmenförmige Seitenteile aufweist, an denen die Verankerungsabschnitte gehalten sind. Die Verankerungsabschnitte sind dann zwischen diesen Seitenteilen praktisch aufgespannt, dies gilt insbesondere bei Verwendung von flächigen, netzförmigen Strukturen.

Die Verankerungsteile können beispielsweise in Bohrungen der rahmenförmigen Seitenteile eintauchen und in diesen festgelegt sein. Zur Festlegung können die Verankerungsteile in die Bohrungen eingeschraubt sein.

Bei einer anderen Ausführungsform ist vorgesehen, daß die Verankerungsteile über Verdickungen oder Knoten an der Außenseite der Seitenteile in den Bohrungen festgelegt sind.

Bei einer weiteren Ausführungsform bestehen die Verankerungsteile aus quellfähigem Material und umschließen beim Quellen Vorsprünge und Rücksprünge in den Bohrungen. Bei Aufnahme von Wasser ergibt sich somit eine formschlüssige Festlegung der Verankerungsteile in den Bohrungen.

Bei einer weiteren bevorzugten Ausführungsform sind die Verankerungsabschnitte U-förmig ausgebildet mit zwei quer zu den Trägern verlaufenden und die Verankerungsabschnitte am Träger festlegenden Schenkeln und einem diese verbindenden Steg. Bei diesen Verankerungsabschnitten läuft der Steg normalerweise im wesentlichen in einer Ebene, die parallel zur Wirbelkörper-Anlagefläche und im Abstand vom Träger angeordnet ist, insbesondere können die Schenkel und der Steg im wesentlichen geradlinig verlaufen.

Es ist dabei günstig, wenn die Schenkel in Bohrungen im Träger eintauchen und in diesen festgelegt sind.

Mehrere benachbarte Verankerungsabschnitte können aus einem einzigen faden- oder stabförmigen Bauteil gebildet sein, das durch entsprechende Abbiegungen oder Abwinklungen eine größere Anzahl von U-förmig ausgebildeten Verankerungsabschnitten ausbildet.

Besonders vorteilhaft ist es, wenn sich überkreuzende Verankerungsteile ein flächiges Bauelement ausbilden, das an seinen Rändern mit dem Träger verbunden ist. In diesem Falle bildet das flächige Bauelement ein selbsttätig handhabbares Bauteil, das als solches mit dem Träger verbunden wird.

Zur Verbindung kann der Träger an seinen Seitenflächen eine umlaufende Bandage tragen, die die Ränder des flächigen Bauteils gegen die Seitenflächen spannt.

Es ist dabei vorteilhaft, wenn die Seitenflächen im Bereich der Bandage eine umlaufende Nut zur Aufnahme der Ränder des flächigen Bauteils aufweisen.

Die Verankerungsabschnitte können entweder von Anfang an in das Material des Kernes eingebettet sein, und dieser Verbundwerkstoff wird dann als Ganzes mit dem Träger verbunden, oder aber es ist vorgesehen, daß die Verankerungsabschnitte mit dem Träger verbunden sind und daß dann auf die Verankerungsabschnitte das Kernmaterial aufgelegt wird, das beim Quellen, also bei Aufnahme von Flüssigkeit, dann die Verankerungsabschnitte umgreift.

Bei bevorzugten Ausführungsformen kann es vorteilhaft sein, wenn die Verankerungsabschnitte biegeschlaff ausgebildet sind, also als biegbare Fäden oder Drähte.

Die Verankerungsabschnitte können aus Metall bestehen, vorzugsweise aus Titan oder einer Titanlegierung.

Zusätzlich kann vorgesehen sein, daß die Verankerungsabschnitte mit einem quellfähigen Material beschichtet sind, so daß beim Umschließen dieser Verankerungsabschnitte eine innige Verbindung zwischen den Verankerungsabschnitten und dem umgebenden Kernmaterial erfolgt. Grundsätzlich wäre es auch möglich, die Verankerungsabschnitte vollständig aus einem quellfähigen Material zu fertigen, so daß beim Quellen des Materials die Verankerungsabschnitte und das umgebende Kernmaterial sich besonders gut miteinander verbinden.

Die Verankerungsabschnitte können auch aus einem hydrophoben Polymer bestehen, beispielsweise aus einem verstreckten Polyethylen, welches in einer besonders bevorzugten Form mit einem hydrophilen, in Wasser quellbaren, aber nicht lösbaren Polymer ummantelt ist.

Bei einer anderen Ausführungsform ist vorgesehen, daß die Verankerungsabschnitte aus einem Hydrogel oder einem Xerogel bestehen.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht von zwei Wirbelkörpern mit einem dazwischen eingesetzten Zwischenwirbelimplantat;
- Figur 2:: eine Endplatte des Zwischenwirbelimplantats der Figur 1 mit einem darauf aufgelegten Kern aus einem quellfähigen Material;
- Figur 3a:: eine Schnittansicht längs Linie 3-3 in Figur 2 vor dem Aufquellen einer quellfähigen Ummantelung eines Verankerungsabschnittes;
- Figur 3b:: eine Ansicht ähnlich Figur 3a nach dem Aufquellen der quellfähigen Ummantelung des Verankerungsabschnittes;
- Figur 4:: eine Schnittansicht längs Linie 4-4 in Figur 2 vor dem Aufquellen des quellfähigen Materials;
- Figur 5:: eine Ansicht ähnlich Figur 4 nach dem Aufquellen des quellfähigen Materials;
- Figur 6:: eine Ansicht ähnlich Figur 2 bei einem Ausführungsbeispiel mit U-förmigen Verankerungsabschnitten;
- Figur 7:: eine Schnittansicht längs Linie 7-7 in Figur 6;
- Figur 8:: eine Ansicht ähnlich Figur 2 mit einem netzförmigen Bauteil aus Verankerungsabschnitten und
- Figur 9:: eine Schnittansicht längs Linie 9-9.

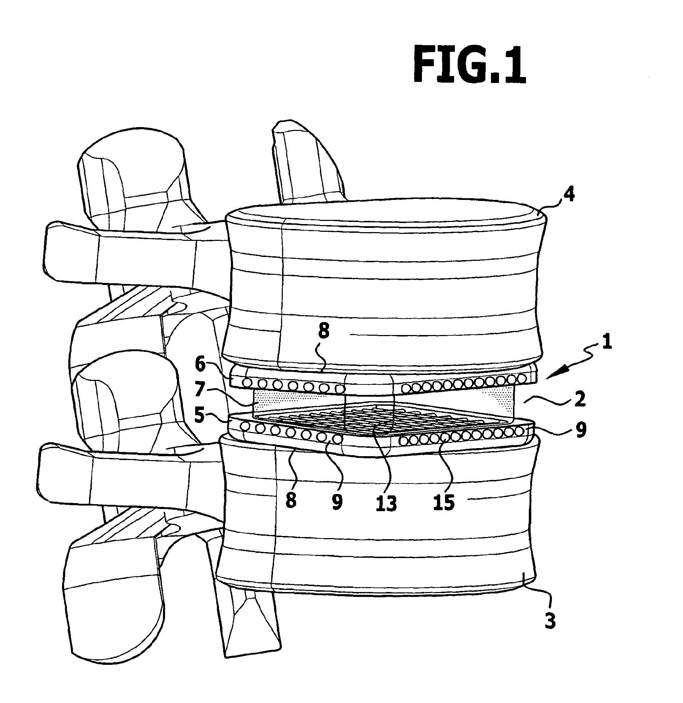

In Figur 1 ist ein Zwischenwirbelimplantat 1 im Zwischenwirbelraum 2 zwischen zwei benachbarten Wirbelkörpern 3, 4 angeordnet. Es umfaßt eine untere Endplatte 5 und eine obere Endplatte 6 und einen zwischen diesen beiden Endplatten 5 und 6 angeordneten Kern 7. Die beiden Endplatten 5, 6 sind spiegelbildlich zueinander ausgebildet, nachfolgend wird daher nur die untere Endplatte 5 näher beschrieben.

Sie umfaßt an ihrer Unterseite eine ebene Anlagefläche 8 und an ihrer Oberseite einen nach oben überstehenden, rahmenförmigen Rand 9, der in sich geschlossen ist und einen Innenraum 10 umschließt, der nach unten durch die Endplatte 5 verschlossen ist und nach oben hin offen ist.

Die Endplatte 5 mit dem nach oben überstehenden Rand 9 besteht aus einem biegefesten und formstabilen, biokompatiblen Material, beispielsweise aus Titan oder einer Titanlegierung oder aber auch aus einem thermoplastischen Kunststoff, beispielsweise aus Polyetheretherketon. Im dargestellten Ausführungsbeispiel ist die Endplatte 5 im wesentlichen rechteckförmig ausgebildet mit abgerundeten Ecken und in ihrer Größe der Querschnittsfläche der Wirbelkörper 3, 4 angepaßt, so daß im wesentlichen der gesamte Zwischenwirbelraum von den Endplatten überdeckt wird.

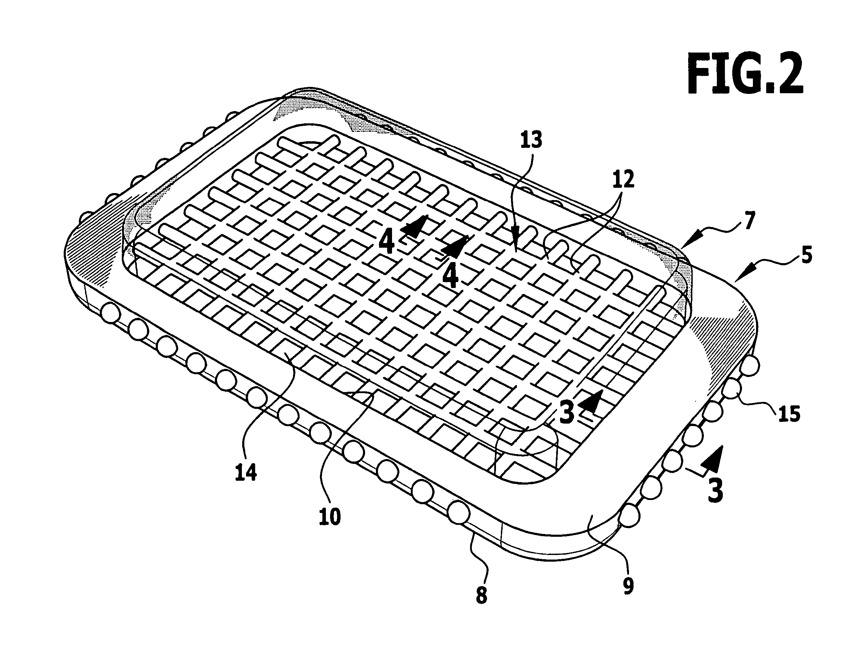

Bei dem in den Figuren 1 bis 3 dargestellten Ausführungsbeispiel sind in dem nach oben überstehenden Rand 9 eine größere Anzahl von quer zur Längsrichtung des Randes 9 verlaufenden Bohrungen 11 angeordnet, die in dem dargestellten Ausführungsbeispiel als durchgehende Innengewindebohrungen ausgeführt sind. In jeweils gegenüberliegende derartige Bohrungen 11 der Endplatte tauchen die Enden von faden- oder drahtförmigen Verankerungsabschnitten 12 ein, die sich auf diese Weise quer durch den gesamten Innenraum 10 erstrecken. Die Verankerungsabschnitte 12 von jeweils gegenüberliegenden Teilen des Randes 9 verlaufen parallel zueinander, und die Verankerungsabschnitte 12 der senkrecht zueinander verlaufenden Abschnitte des Randes 9 verlaufen dementsprechend senkrecht zueinander, das heißt sie überkreuzen sich und bilden somit eine netzartige Verankerungsfläche 13 aus. Diese erstreckt sich parallel zur Endplatte 5 im Abstand von deren Oberseite 14 und im Innenraum 10. Dabei wird der gesamte Innenraum gleichmäßig von den Verankerungsabschnitten 12 überdeckt, wie dies aus der Darstellung der Figur 2 zu entnehmen ist.

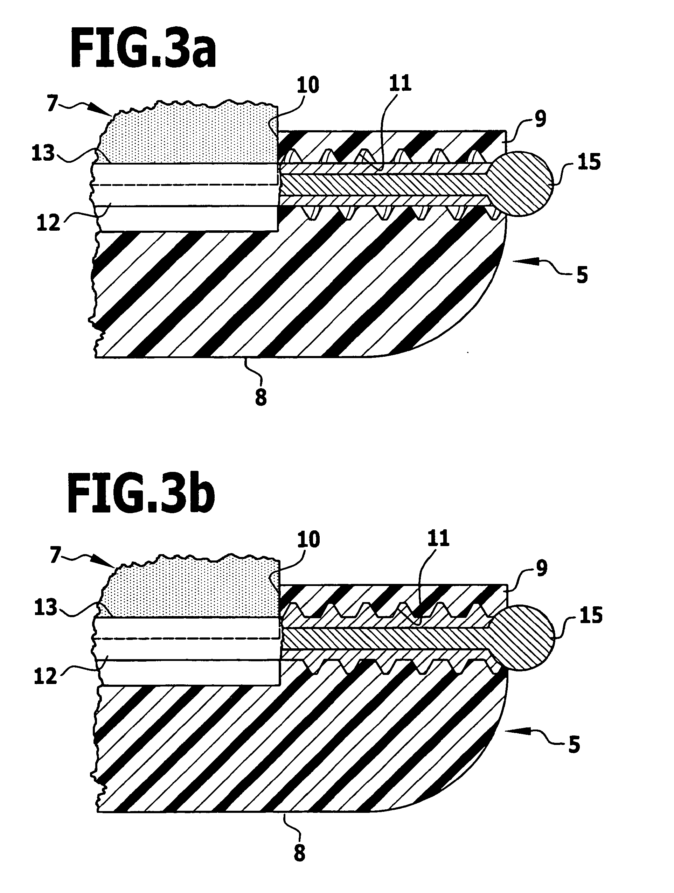

Die Verankerungsabschnitte 12 sind mit ihren in die Bohrungen 11 eintauchenden Enden fest mit dem Rand 9 verbunden. Wie aus den Figuren 3a und 3b zu entnehmen ist, können die Verankerungsabschnitte 12 eine Beschichtung aus quellfähigem Material aufweisen, welches unter Wasseraufnahme sein Volumen vergrößert. Es kann sich dabei nur um die Ummantelung handeln, es kann aber auch vorgesehen sein, daß der Verankerungsabschnitt vollständig aus einem solchen Material besteht. Die Enden der in dieser Weise ausgebildeten Verankerungsabschnitte 12 sind im Bereich des Randes 9 in einer Innengewindebohrung angeordnet. Vor dem Aufquellen der quellfähigen Ummantelung tritt das quellfähige Material aufgrund seines geringen Volumens nicht in die Gewindegänge der Innengewindebohrung ein (Figur 3a), das heißt der Außendurchmesser der Verankerungsabschnitte entspricht dem kleinsten Innendurchmesser der Innengewindebohrung, so daß die Verankerungsabschnitte in die Innengewindebohrung eingeschoben werden können.

Nach Flüssigkeitsaufnahme vergrößert sich das Volumen des quellfähigen Mantelmaterials oder des quellfähigen Materials der Verankerungsabschnitte derart, daß dieses Material in die Gewindegänge eintritt und diese mehr oder weniger vollständig ausfüllt, wie dies in Figur 3b dargestellt ist. Dadurch ergibt sich eine formschlüssige Verankerung der Verankerungsabschnitte im Rand 9.

Die Festlegung der Verankerungsabschnitte 12 im Rand 9 kann aber auch ausschließlich oder zusätzlich durch Verdickungen 15 erfolgen, die an der Außenseite des Randes 9 anliegen und die Verankerungsabschnitte 12 zwischen den gegenüberliegenden Teilen des Rahmens 9 aufspannen.

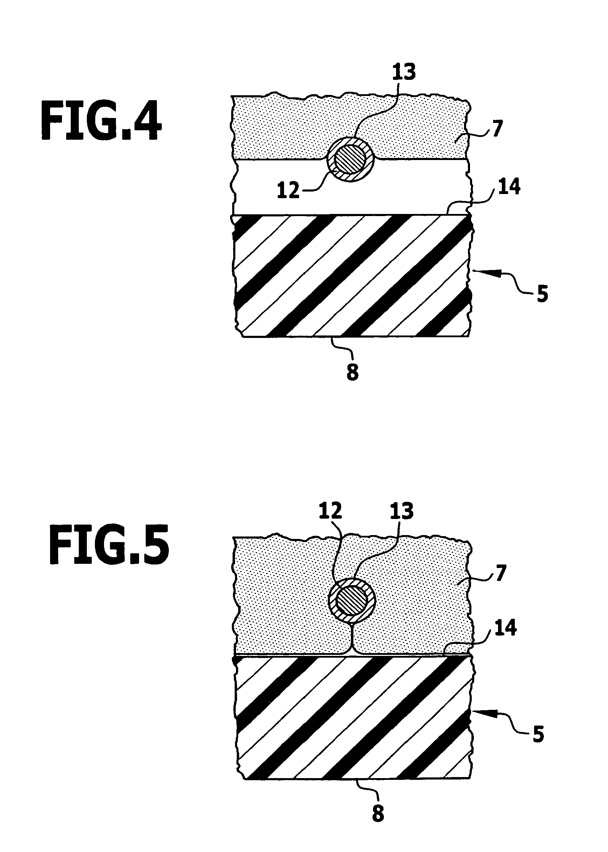

Die Verankerungsabschnitte 12 bestehen aus einem Material in Form eines Fadens, insbesondere eines biegeschlaffen Fadens oder in Form eines Drahtes oder eines sehr dünnen, länglichen Stabes, so daß zwischen der Oberseite 14 der Endplatte 5 und der Unterseite der Verankerungsabschnitte 12 ein Abstand verbleibt (Figur 4).

Die Verankerungsabschnitte können vorzugsweise aus Metall bestehen, insbesondere aus Titan oder einer Titanlegierung, es sind aber auch andere Materialien verwendbar, beispielsweise hydrophobe Polymere wie verstrecktes Polyethylen, bevorzugt hochmolekulares Polyethylen, insbesondere ultrahochmolekulares Polyethylen (UHMWPE) oder nicht vollständig gequollenes Hydrogel, oder die Verankerungsabschnitte können aus einem Hydrogel oder einem Xerogel bestehen, also einem reinen, wasserfreien Polymer. Die Verankerungsabschnitte 12 können als Multifilament oder Monofilament ausgebildet sein, und es ist möglich, daß die Verankerungsabschnitte 12 zusätzlich beschichtet sind, beispielsweise mit einem quellfähigen Xerogel, welches bei Kontakt mit der vorhandenen Körperflüssigkeit ein Hydrogel ausbildet.

Auf die von den Verankerungsabschnitten 12 ausgebildete Verankerungsfläche 13 ist der plattenförmige, in seinem Querschnitt dem Querschnitt des Innenraumes 10 angepaßte Kern aufgelegt, so daß dessen Unterseite auf den Verankerungsabschnitten 12 aufliegt. An der Oberseite des Zwischenwirbelimplantates 1 kann die gleich aufgebaute obere Endplatte 6 aufgelegt werden, die sich dann mit ihren Verankerungsabschnitten 12 an der Oberseite des Kerns 7 abstützt.

Der Kern besteht aus einem quellfähigen Hydrogel, also einem Material, das unter Flüssigkeitsaufnahme sein Volumen vergrößern kann.

Als Hydrogele kommen prinzipiell alle nicht degradierbaren hydrophilen Polymere in Frage. Beispiele sind Polyacrylsäure und deren Derivate wie Polymethacrylsäure, Polyacrylsäureamid, Polyacrylonitril, Polyacrylsäureester, Polyhydroxyethylmethacrylate, außerdem Polyvinylpyrrolidon (PVP), Polyurethane, hochmolekularer Polyvinylalkohol.

Denkbar sind auch Polymerblends (Copolymere, welche über kovalente Bindungen miteinander verbunden sind) aus den oben genannten Polymeren oder Interpenetrating Networks (IPN) aus den oben genannten Polymeren. IPN bestehen aus mindestens zwei unterschiedlichen Polymeren, deren Polymerketten ineinander verhakt sind und über physikalische Wechselwirkungen (van der Waals-, elektrostatische, H-Brückenbindungen und / oder ionische Kräfte) miteinander verbunden sind.

Weitere Polymermischungen, welche eingesetzt werden können, sind Copolymer sowie IPN aus Polyacrylaten (Polyacrylsäure und deren Derivate wie Polymethacrylsäure, Polyacrylsäureamid, Polyacrylsäurenitril, Polyacrylsäureester) mit Polycaprolacton.

Das quellfähige Material des Kernes 7 vergrößert bei Flüssigkeitsaufnahme, das heißt nach der Implantation in den Zwischenwirbelraum 2, sein Volumen, und dabei umschließt das Material des Kernes 7 die Verankerungsabschnitte 12 der Verankerungsfläche 13 allseits, das heißt der gesamte Innenraum 10 der beiden Endplatten 5, 6 wird vollständig ausgefüllt, wie dies in Figur 5 dargestellt ist. Dadurch erfolgt eine Einbettung der Verankerungsabschnitte 12 in das Material des Kernes 7 und damit eine verzahnende Verbindung des Kernes mit den benachbarten Endplatten.

Diese Verbindung kann noch dadurch verbessert werden, daß zwischen dem Material der Verankerungsabschnitte und dem Material des Kernes nicht nur ein Formschluß ausgebildet wird, sondern daß zusätzlich flexible elektrostatische, ionische oder van-der-Waals-Wechselwirkungen zur Verankerung des Kernes mit den Verankerungsabschnitten genutzt werden. Dabei lassen sich die Eigenschaften des Materials des Kernes 7, insbesondere die Quellfähigkeit, durch unterschiedliche Parameter des quellfähigen Materials in gewissen Grenzen variieren, beispielsweise durch die Verwendung eines unterschiedlichen Molekulargewichts, durch einen unterschiedlichen Vernetzungsgrad oder durch ein unterschiedliches Herstellungsverfahren.

Insbesondere ergibt sich eine besonders gute Vernetzung bei Verankerungsabschnitten 12, die ihrerseits mit einem Polymer beschichtet sind, dabei kann es sich um dasselbe polymere Material handeln, wie beim Material des Kernes, es können aber auch unterschiedliche polymere Materialien eingesetzt werden, die miteinander eine innige Vernetzung eingehen.

Bei dem Ausführungsbeispiel der Figuren 1 bis 5 liegen die Verankerungsabschnitte 12 in Form einer netzartigen Verankerungsfläche 13 vor.

Es sind auch andere geometrische Anordnungen der Verankerungsabschnitte 12 auf den Endplatten 5, 6 möglich, wichtig ist lediglich, daß dem quellfähigen Material die Möglichkeit gegeben wird, die Verankerungsabschnitte allseitig zu umschließen.

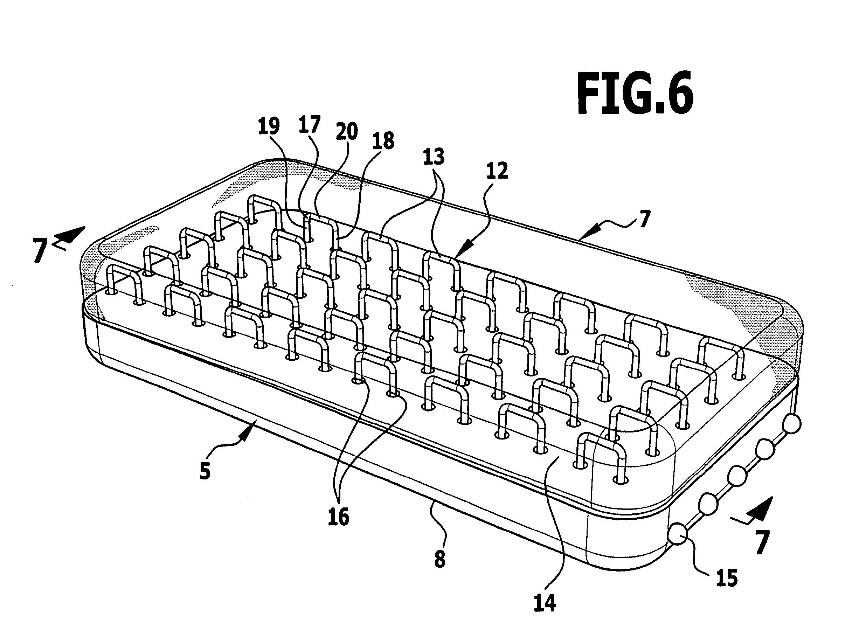

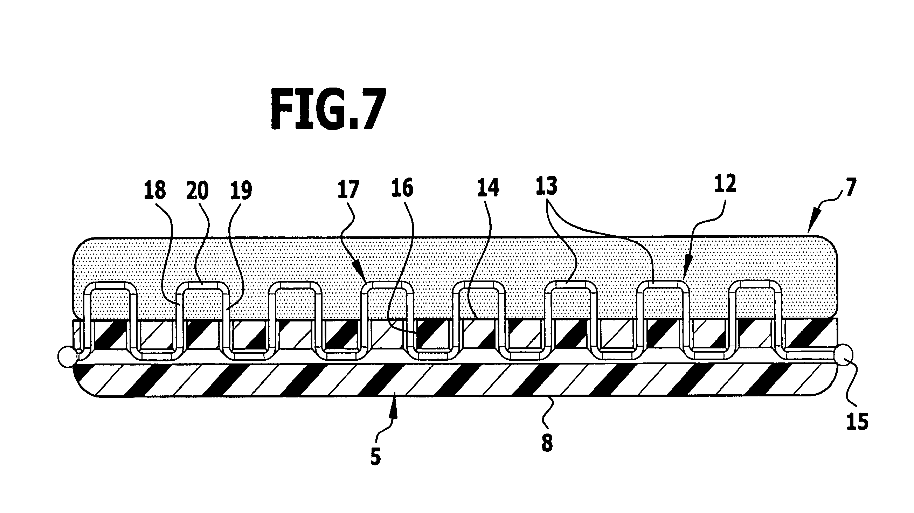

Bei dem Ausführungsbeispiel der Figuren 6 und 7 wird ein in die Endplatte 5 eingebettetes, drahtförmiges Bauelement durch nebeneinanderliegende Bohrungen 16 in der Oberseite 14 der Endplatte 5 so schlaufenförmig nach oben und wieder nach unten geführt, daß oberhalb der Oberseite 14 der Endplatte 5 im Querschnitt U-förmige Brücken oder Schlaufen 17 entstehen mit aus den Bohrungen 16 senkrecht nach oben austretenden parallelen Schenkeln 18, 19 und diese verbindenden, parallel zur Oberseite 14 und im Abstand von dieser verlaufenden Stegen 20. Eine größere Anzahl derartiger Reihen von Schlaufen 17 sind an der Oberseite 14 der Endplatte 5 nebeneinander angeordnet und überdecken dadurch die gesamte Oberseite 14. Die parallel zur Oberseite 14 verlaufenden Stege 20 bilden dabei eine Verankerungsfläche 13 aus, auf die der Kern 7 in ähnlicher Weise wie im Ausführungsbeispiel der Figuren 1 bis 5 aufgelegt wird. Beim Quellen umschließt das Material des Kernes dann die Stege 20 allseits und auch die aus der Oberseite 14 vorstehenden Schenkel 18, 19, so daß eine feste und sichere Verbindung zwischen dem Kern 7 und der Endplatte 5 gewährleistet ist.

Die schlaufenförmigen Bauelemente können als Drahtelemente ausgebildet sein, also aus einem metallischen Werkstoff bestehen, insbesondere aus Titan oder einer Titanlegierung, es kann sich dabei aber auch um Polymerfäden handeln, die in diesem Falle vorzugsweise biegesteif ausgebildet sind, beispielsweise um Polyethylenfäden.

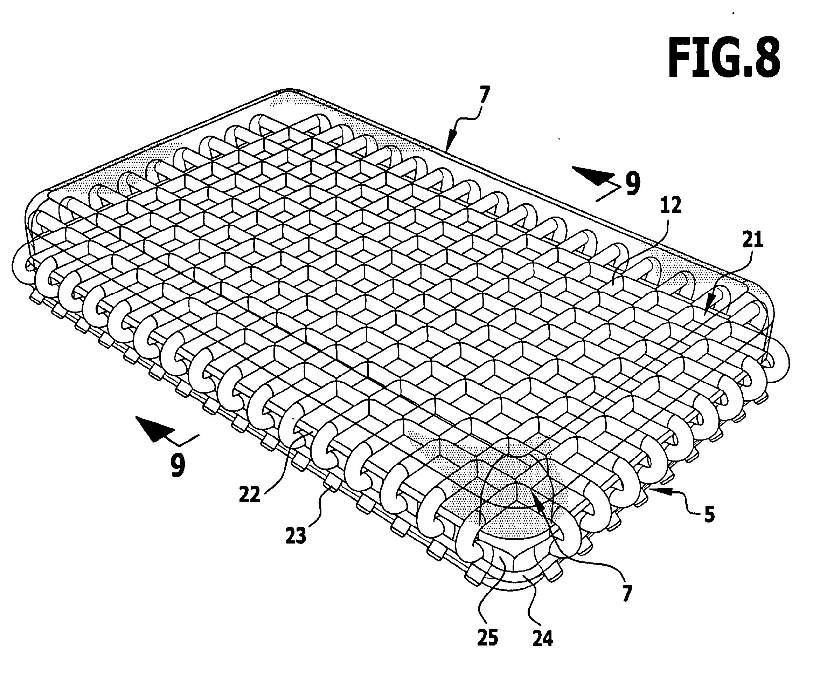

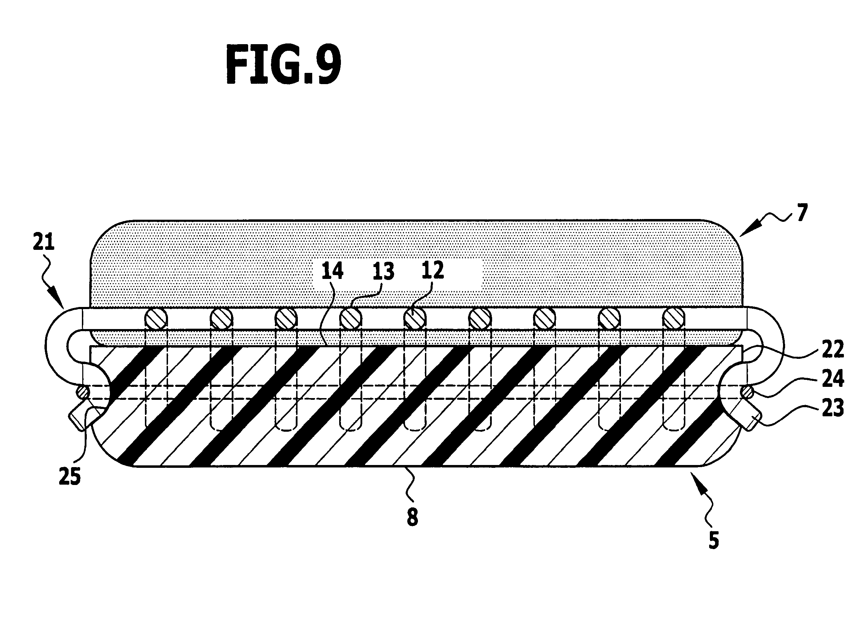

Eine weitere mögliche Ausgestaltung ist in dem Ausführungsbeispiel der Figuren 8 und 9 dargestellt. Dort sind parallel zueinander verlaufende und sich kreuzende Verankerungsabschnitte 12 zu einer Verankerungsfläche 13 kombiniert, die als selbständig handhabbares Bauteil ausgebildet ist. Zu diesem Zweck können die Verankerungsabschnitte beispielsweise verwebt sein oder an den Kreuzungspunkten in geeigneter Weise miteinander verbunden sein. Im Randbereich wird dieses selbsttätig handhabbare, netzartige Bauteil 21 mit nach unten abgebogenen Rändern ausgebildet, das heißt die Enden der Verankerungsabschnitte 12 werden nach unten gebogen, so daß sie von außen an den Seitenflächen 20 der Endplatte 5 zur Anlage kommen, wenn das Bauteil 21 die Endplatte 5 überdeckend auf diese aufgelegt wird (Figur 8). Die Enden 23 der Verankerungsabschnitte 12 werden dann durch eine umlaufende Bandage 24 fest gegen die Seitenflächen 22 gedrückt, so daß dadurch das Bauteil 21 an der Endplatte 5 dauerhaft festgelegt wird. Die Fixierung wird dadurch unterstützt, daß in der Seitenfläche 22 eine umlaufende Nut 25 eingearbeitet ist, die sich längs der Seitenflächen 22 um die gesamte Endplatte 5 herum erstreckt und in die die Enden 23 durch die Bandage 24 hineingedrückt werden (Figur 9).

Auf das Bauteil 21 ist in ähnlicher Weise wie bei den voranstehend beschriebenen Ausführungsbeispielen der Kern 7 aufgelegt, der dann beim Quellen die Verankerungsabschnitte 12 allseits umgibt und zu einer festen Verbindung mit der Endplatte 5 kommt.

Grundsätzlich wäre es auch möglich, den Kern 7 bereits vor der Montage des Bauteils 21 an der Endplatte 5 mit dem Bauteil 21 zu verbinden, also das Bauteil 21 in den Kern 7 schon bei der Herstellung einzubetten. Ein Kern mit in dieser Weise eingebettetem Bauteil 21 kann dann auf eine Endplatte 5 aufgesetzt und durch die Bandage 24 an der Endplatte 5 festgelegt werden. Damit erhält man bereits eine Verbindung vor dem Quellen des quellfähigen Materials im Körper.

## Patentansprüche

1. Zwischenwirbelimplantat (1) mit mindestens einem eine Wirbelkörper-Anlagefläche (8) aufweisenden Anlageelement und mit einem quellfähigen Kern (7), der auf der der Wirbelkörper-Anlagefläche (8) abgewandten Seite mit dem Anlageelement verbunden ist, wobei das Anlageelement einen Träger (5) umfaßt, auf dessen einer Seite die Wirbelkörper-Anlagefläche (8) angeordnet ist und der auf seiner anderen Seite Vorsprünge trägt, die von dem Material des Kernes (7) umschlossen werden, **dadurch gekennzeichnet, daß** die Vorsprünge längliche, faden- oder stabförmige Verankerungsabschnitte (12) aufweisen, die im Abstand vom Träger (5) verlaufen und allseits frei zugänglich sind.

2. Implantat nach Anspruch 1, **dadurch gekennzeichnet, daß** die Verankerungsabschnitte (12) im wesentlichen parallel zur Wirbelkörper-Anlagefläche (8) und im Abstand von der der Wirbelkörper-Anlagefläche (8) abgewandten Seite (14) des Trägers (5) verlaufen.

3. Implantat nach Anspruch 1 oder 2, **dadurch gekennzeichnet, daß** im wesentlichen die gesamte Fläche des Trägers (5) Verankerungsabschnitte (12) aufweist.

4. Implantat nach einem der Ansprüche 1 bis 3, **dadurch gekennzeichnet, daß** mehrere Verankerungsabschnitte (12) parallel zueinander verlaufen.

5. Implantat nach einem der Ansprüche 1 bis 3, **dadurch gekennzeichnet, daß** sich verschiedene Verankerungsabschnitte (12) überkreuzen.

6. Implantat nach Anspruch 5, **dadurch gekennzeichnet, daß** sich überkreuzende Verankerungsabschnitte (12) eine netzförmige Struktur (13; 21) ausbilden.

7. Implantat nach einem der voranstehenden Ansprüche, **dadurch gekennzeichnet, daß** der Träger (5) rahmenförmige Seitenteile (9) aufweist, an denen die Verankerungsabschnitte (12) gehalten sind.

8. Implantat nach Anspruch 7, **dadurch gekennzeichnet, daß** die Verankerungsteile (12) in Bohrungen (11) der rahmenförmigen Seitenteile (9) eintauchen und in diesen festgelegt sind.

9. Implantat nach Anspruch 8, **dadurch gekennzeichnet, daß** die Verankerungsteile (12) in die Bohrungen (11) eingeschraubt sind.

10. Implantat nach Anspruch 8, **dadurch gekennzeichnet, daß** die Verankerungsteile (12) über Verdickungen (15) oder Knoten an der Außenseite der Seitenteile (9) in den Bohrungen (11) festgelegt sind.

11. Implantat nach Anspruch 8, **dadurch gekennzeichnet, daß** die Verankerungsteile (12) aus quellfähigem Material bestehen und beim Quellen Vorsprünge und Rücksprünge in den Bohrungen (11) umschließen.

12. Implantat nach einem der Ansprüche 1 bis 5, **dadurch gekennzeichnet, daß** die Verankerungsabschnitte (12) U-förmig ausgebildet sind mit zwei quer zu dem Träger (5) verlaufenden und die Verankerungsabschnitte (12) am Träger (5) festlegenden Schenkeln (18, 19) und einem diese verbindenden Steg (20).

13. Implantat nach Anspruch 12, **dadurch gekennzeichnet, daß** die Schenkel (18, 19) und der Steg (20) im wesentlichen geradlinig verlaufen.

14. Implantat nach Anspruch 12 oder 13, **dadurch gekennzeichnet, daß** die Schenkeln (18, 19) in Bohrungen (16) im Träger (5) eintauchen und in diesen festgelegt sind.

15. Implantat nach einem der Ansprüche 12 bis 14, **dadurch gekennzeichnet, daß** mehrere benachbarte Verankerungsabschnitte (12) aus einem einzigen faden- oder stabförmigen Bauteil gebildet werden.

16. Implantat nach einem der Ansprüche 5 oder 6, **dadurch gekennzeichnet, daß** sich überkreuzende Verankerungsteile (12) ein flächiges Bauelement (21) ausbilden, das an seinen Rändern mit dem Träger (5) verbunden ist.

17. Implantat nach Anspruch 16, **dadurch gekennzeichnet, daß** der Träger (5) an seinen Seitenflächen (22) eine umlaufende Bandage (24) trägt, die die Ränder des flächigen Bauteils (21) gegen sie Seitenflächen (22) spannt.

18. Implantat nach Anspruch 17, **dadurch gekennzeichnet, daß** die Seitenflächen (22) im Bereich der Bandage (24) eine umlaufende Nut (25) zur Aufnahme der Ränder des flächigen Bauteils (21) aufweisen.

19. Implantat nach einem der voranstehenden Ansprüche, **dadurch gekennzeichnet, daß** die Verankerungsabschnitte (12) biegeschlaff ausgebildet sind.

20. Implantat nach einem der voranstehenden Ansprüche, **dadurch gekennzeichnet, daß** die Verankerungsabschnitte (12) aus Metall bestehen.

21. Implantat nach Anspruch 20, **dadurch gekennzeichnet, daß** die Verankerungsabschnitte (12) aus Titan oder einer Titanlegierung bestehen.

22. Implantat nach Anspruch 20 oder 21, **dadurch gekennzeichnet, daß** die Verankerungsabschnitte (12) mit einem quellfähigen Material beschichtet sind.

23. Implantat nach einem der Ansprüche 1 bis 19, **dadurch gekennzeichnet, daß** die Verankerungsabschnitte (12) aus einem hydrophoben Polymer bestehen.

24. Implantat nach Anspruch 23, **dadurch gekennzeichnet, daß** die Verankerungsabschnitte (12) aus Polyethylen bestehen.

25. Implantat nach einem der Ansprüche 1 bis 19, **dadurch gekennzeichnet, daß** die Verankerungsabschnitte (12) aus einem Hydrogel oder einem Xerogel bestehen.

## Claims

1. Intervertebral implant (1) with at least one contact element having a vertebral body contact face (8) and with a swellable core (7), which is connected to the contact element on the side remote from the vertebral body contact face (8), wherein the contact element comprises a support (5), on one side of which the vertebral body contact face (8) is arranged and which on its other side carries projections surrounded by the material of the core (7), **characterised in that** the projections have anchoring sections (12), which are elongated, thread-like or rod-like, and which extend at a spacing from the support (5) and are freely accessible from all sides.

2. Implant according to Claim 1, **characterised in that** the anchoring sections (12) extend substantially parallel to the vertebral body contact face (8) and at a spacing from the side (14) of the support (5) remote from the vertebral body contact face (8).

3. Implant according to Claim 1 or 2, **characterised in that** substantially the entire surface of the support (5) has anchoring sections (12).

4. Implant according to one of Claims 1 to 3, **characterised in that** a plurality of anchoring sections (12) extend parallel to one another.

5. Implant according to one of Claims 1 to 3, **characterised in that** various anchoring sections (12) intersect.

6. Implant according to Claim 5, **characterised in that** intersecting anchoring sections (12) form a net-like structure (13; 21).

7. Implant according to one of the preceding claims, **characterised in that** the support (5) has frame-like side parts (9), on which the anchoring sections (12) are held.

8. Implant according to Claim 7, **characterised in that** the anchoring parts (12) penetrate into holes (11) of the frame-like side parts (9) and are secured in these.

9. Implant according to Claim 8, **characterised in that** the anchoring parts (12) are screwed into the holes (11).

10. Implant according to Claim 8, **characterised in that** the anchoring parts (12) are secured in the holes (11) by means of enlargements (15) or knots on the outside of the side parts (9).

11. Implant according to Claim 8, **characterised in that** the anchoring parts (12) are made of swellable material and surround processes and recesses in the holes upon swelling.

12. Implant according to one of Claims 1 to 5, **characterised in that** the anchoring sections (12) are U-shaped with two legs (18, 19), which extend transversely to the support (5) and secure the anchoring sections (12) on the support (5), and with a web (20) connecting these legs.

13. Implant according to Claim 12, **characterised in that** the legs (18, 19) and the web (20) extend substantially rectilinearly.

14. Implant according to Claim 12 or 13, **characterised in that** the legs (18, 19) penetrate into holes (16) in the support (5) and are secured in these.

15. Implant according to one of Claims 12 to 14, **characterised in that** a plurality of adjacent anchoring sections (12) are formed from a single thread- or rod-like structural part.

16. Implant according to one of Claims 5 or 6, **characterised in that** intersecting anchoring parts (12) form a flat structural element (21), which is connected to the support (5) at its edges.

17. Implant according to Claim 16, **characterised in that** on its side faces (22) the support (5) carries a peripheral band (24), which clamps the edges of the flat structural part (21) against the side faces (22).

18. Implant according to Claim 17, **characterised in that** in the region of the band (24) the side faces (22) have a peripheral groove (25) to receive the edges of the flat structural part (21).

19. Implant according to one of the preceding claims, **characterised in that** the anchoring sections (12) are pliable.

20. Implant according to one of the preceding claims, **characterised in that** the anchoring sections (12) are made of metal.

21. Implant according to Claim 20, **characterised in that** the anchoring sections (12) are made of titanium or a titanium alloy.

22. Implant according to Claim 20 or 21, **characterised in that** the anchoring sections (12) are coated with a swellable material.

23. Implant according to one of Claims 1 to 19, **characterised in that** the anchoring sections (12) are made of a hydrophobic polymer.

24. Implant according to Claim 23, **characterised in that** the anchoring sections (12) are made of polyethylene.

25. Implant according to one of Claims 1 to 19, **characterised in that** the anchoring sections (12) are made of a hydrogel or a xerogel.

## Revendications

1. Implant intervertébral (1) comprenant au moins un élément d'appui présentant une surface d'appui de vertèbre (8), et comprenant un noyau (7) susceptible de gonflement, qui, sur le côté opposé à celui où se trouve la surface d'appui de vertèbre (8), est relié à l'élément d'appui, l'élément d'appui englobant un support (5) sur un côté duquel est agencée la surface d'appui de vertèbre (8) et qui sur son autre côté porte des protubérances entourées par le matériau du noyau (7),
**caractérisé en ce que** les protubérances présentent des tronçons d'ancrage (12) allongés, en forme de fil ou de tige, qui s'étendent à distance du support (5) et sont librement accessibles de tous côtés.

2. Implant selon la revendication 1, **caractérisé en ce que** les tronçons d'ancrage (12) s'étendent sensiblement de manière parallèle à la surface d'appui de vertèbre (8) et à distance du côté (14) du support (5), qui est opposé au côté présentant la surface d'appui de vertèbre (8).

3. Implant selon la revendication 1 ou 2, **caractérisé en ce que** sensiblement toute la surface du support (5) présente des tronçons d'ancrage (12).

4. Implant selon l'une des revendications 1 à 3, **caractérisé en ce que** plusieurs tronçons d'ancrage (12) s'étendent parallèlement les uns aux autres.

5. Implant selon l'une des revendications 1 à 3, **caractérisé en ce que** différents tronçons d'ancrage (12) se croisent mutuellement.

6. Implant selon la revendication 5, **caractérisé en ce que** des tronçons d'ancrage (12), qui se croisent, forment une structure en forme de treillis (13; 21).

7. Implant selon l'une des revendications précédentes, **caractérisé en ce que** le support (5) présente des parties latérales (9) en forme de cadre, sur lesquelles sont maintenus les tronçons d'ancrage (12).

8. Implant selon la revendication 7, **caractérisé en ce que** les tronçons d'ancrage (12) s'engagent dans des perçages (11) des parties latérales (9) en forme de cadre, et sont fixés dans ceux-ci.

9. Implant selon la revendication 8, **caractérisé en ce que** les tronçons d'ancrage (12) sont vissés dans les perçages (11).

10. Implant selon la revendication 8, **caractérisé en ce que** les tronçons d'ancrage (12) sont fixés dans les perçages (11), par l'intermédiaire de renflements (15) ou de noeuds sur le côté extérieur des parties latérales (9).

11. Implant selon la revendication 8, **caractérisé en ce que** les tronçons d'ancrage (12) sont réalisés en un matériau susceptible de gonflement, et, lors du gonflement, entourent des protubérances ou des retraits dans les perçages (11).

12. Implant selon l'une des revendications 1 à 5, **caractérisé en ce que** les tronçons d'ancrage (12) sont de configuration en forme de U et comportent d'une part deux ailes (18, 19), qui s'étendent transversalement au support (5) et fixent les tronçons d'ancrage (12) au support (5), et d'autre part une branche (20) qui relie ces ailes.

13. Implant selon la revendication 12, **caractérisé en ce que** les ailes (18, 19) et la branche (20) s'étendent sensiblement de manière rectiligne.

14. Implant selon la revendication 12 ou 13, **caractérisé en ce que** les ailes (18, 19) s'engagent dans des perçages (16) du support (5) et y sont fixées.

15. Implant selon l'une des revendications 12 à 14, **caractérisé en ce que** plusieurs tronçons d'ancrage (12) voisins sont formés par une seule pièce en forme de fil ou de tige.

16. Implant selon l'une des revendications 5 ou 6, **caractérisé en ce que** des tronçons d'ancrage (12) qui se croisent forment un élément plan surfacique (21), qui, au niveau de ses bords, est relié au support (5).

17. Implant selon la revendication 16, **caractérisé en ce que** le support (5) porte sur ses surfaces latérales (22), un bandage (24) périphérique qui serre les bords de l'élément plan surfacique (21) contre les surfaces latérales (22).

18. Implant selon la revendication 17, **caractérisé en ce que** les surfaces latérales (22) présentent dans la zone du bandage (24), une rainure périphérique (25) destinée à recevoir les bords de l'élément plan surfacique (21).

19. Implant selon l'une des revendications précédentes, **caractérisé en ce que** les tronçons d'ancrage (12) sont d'une configuration souple en flexion.

20. Implant selon l'une des revendications précédentes, **caractérisé en ce que** les tronçons d'ancrage (12) sont réalisés en métal.

21. Implant selon la revendication 20, **caractérisé en ce que** les tronçons d'ancrage (12) sont réalisés en titane ou en un alliage de titane.

22. Implant selon la revendication 20 ou 21, **caractérisé en ce que** les tronçons d'ancrage (12) sont revêtus d'un matériau susceptible de gonflement.

23. Implant selon l'une des revendications 1 à 19, **caractérisé en ce que** les tronçons d'ancrage (12) sont réalisés en un polymère hydrophobe.

24. Implant selon la revendication 23, **caractérisé en ce que** les tronçons d'ancrage (12) sont réalisés en polyéthylène.

25. Implant selon l'une des revendications 1 à 19, **caractérisé en ce que** les tronçons d'ancrage (12) sont réalisés en un hydrogel ou xerogel.
